# FASCICULE DE BREVET EUROPEEN

(11) **EP 1 957 011 B1**
(45) Date de publication et mention de la délivrance du brevet: **20.09.2017**
(21) Numéro de dépôt: 06841853.2
(22) Date de dépôt: 04.12.2006
(51) Int. Cl.: A61F 2/44

(54) **CAGES DE CONTENTION ET DE FUSION INTERSOMATIQUE DES VERTEBRES**
GEHÄUSE ZUR EINSTELLUNG UND INTERSOMATISCHEN FUSION VON WIRBELN
CAGES FOR SETTING AND INTERSOMATICALLY FUSING VERTEBRAE

(30) Priorité: 05.12.2005 FR 0512308
(43) Date de publication de la demande: 20.08.2008
(73) Titulaire: Spineart SA, 1217 Meyrin (CH)
(72) Inventeur: LEVIEUX, Jérôme, 1293 Bellevue (CH)
(74) Mandataire: Cabinet Plasseraud
(86) Numéro de dépôt international: PCT/FR2006/002644
(87) Numéro de publication internationale: WO 2007/065993

(56) Documents cités:
- EP-A- 1 374 809
- EP-A- 1 470 803
- WO-A-03/005938
- US-A- 5 423 816
- US-A- 5 683 394
- US-A1- 2004 193 269
- US-A1- 2005 065 605

## Description

La présente invention concerne des cages de contention et de fusion intersomatique des vertèbres et ses applications, ainsi qu'un dispositif de démontage.

Les systèmes de contention et de fusion intersomatique des vertèbres sont utilisés lors de la chirurgie du rachis cervical. Ces systèmes reposent sur l'utilisation d'une prothèse, appelée cage intervertébrale, pour rétablir la distance anatomique entre deux vertèbres adjacentes lorsque le disque intervertébral est affaissé. La cage permet d'opérer une immobilisation relative de ces vertèbres par croissance d'un greffon osseux installé dans la cage, dans l'espace intervertébral.

FR-A-2 851 457, 2 736 538, 2 703 580, 2 816 201, 2 808 673 et 2 790 945 par exemple décrivent des systèmes de contention et de fusion intersomatique des vertèbres comportant au moins une cage.

Un type d'implant perfectionné est la cage-plaque décrite dans le brevet FR-A-2 747 034. Ce système comporte une cage interne ouverte recevant de l'os spongieux qui est destinée à être interposée entre deux vertèbres et qui permet la mise en place d'un greffon osseux ou d'un matériau favorisant la fusion intersomatique pour induire la fusion des deux vertèbres concernées. La cage décrite dans ce système comporte sur sa face antérieure un élément externe formant une plaque s'étendant dans un plan sensiblement perpendiculaire au plan d'introduction de la cage de part et d'autre de celle-ci, et disposant à chacune de ses extrémités de moyens d'ancrage sur au moins deux vertèbres adjacentes à solidariser entre elles. Ce dispositif permet d'assurer la position et la non-mobilité de la cage. La plaque externe peut être obtenue de manière monobloc avec la cage interne ou ces deux éléments peuvent être réalisés distinctement et solidarisés entre eux ultérieurement par l'intermédiaire de moyens d'assemblage. Ces moyens d'assemblages conduisent à un « verrouillage » de l'ensemble cage-plaque, qui ne permet pas un démontage aisé par le chirurgien. En effet, la plaque, lorsqu'elle est démontable, doit être translatée parallèlement au plan de la face antérieure de la cage, et les dernières techniques chirurgicales dites « mini-invasives » ne laissent pas de place autour de ladite plaque pour permettre une telle translation.

Or, il arrive que la présence permanente d'une plaque sur la partie antérieure des corps vertébraux cervicaux gêne le patient.

US 2004/0193269 décrit un système de contention et de fusion Intersomatique des vertèbres comportant au moins une cage et une plaque agencée perpendiculairement au plan général de ladite cage, dans lequel ladite plaque et ladite cage, fixes l'une par rapport à l'autre, possèdent un trou et un téton pouvant s'insérer et se bloquer de manière réversible dans ledit trou, ladite plaque pouvant être ôtée par simple traction.

Il serait donc souhaitable de disposer d'un système de contention et de fusion intersomatique des vertèbres, qui permettrait de pouvoir facilement retirer la plaque en laissant la cage en place, notamment sans avoir à procéder préalablement à sa translation.

Il serait aussi important que le chirurgien puisse choisir l'emplacement où implanter les vis de fixation de la plaque et dans le cas où il aurait échoué l'implantation d'une vis, il puisse choisir un autre emplacement pour réimplanter une autre vis.

Il serait donc souhaitable de disposer d'un système de contention et de fusion intersomatique des vertèbres, qui permettrait au chirurgien de positionner librement la plaque sur les corps vertébraux.

Or après de longues recherches la demanderesse a mis au point une cage dans laquelle le démontage de la cage-plaque est facilité. De plus, dans certains modes de réalisation, la plaque peut tourner librement tant que les vis ne sont pas implantées dans les corps vertébraux.

C'est pourquoi la présente demande a pour objet un système de contention et de fusion intersomatique des vertèbres comportant au moins une cage et une plaque agencée perpendiculairement au plan général de ladite cage, caractérisé en ce que ladite plaque et ladite cage possèdent l'une un trou et l'autre un téton pouvant s'insérer et se bloquer de manière réversible dans ledit trou, ladite plaque pouvant être ôtée par simple traction.

Une cage peut présenter une forme variée, notamment cylindrique, tronconique ou parallélépipédique et est de préférence réalisée en. Titane, PEEK (polyétheréthercétone) ou tout matériau bio-compatible. Elle comporte des faces latérales antérieure et postérieure ainsi que gauche et droite déterminant l'écartement intervertébral, ainsi que des faces supérieure et inférieure ouvertes.

La plaque présente de préférence une forme parallélépipédique ou oblongue généralement plane. Elle est avantageusement allongée et notamment réalisée en titane, inox ou tout matériau bio-compatible.

Pour assurer sa fixation sur des vertèbres adjacentes, la plaque possède, en général vers chacune de ses deux extrémités, un trou permettant la fixation de ladite plaque avec des vis implantées dans les corps vertébraux.

Selon un mode de réalisation de l'invention, la cage comporte le téton tandis que la plaque comporte le trou. Mais selon un mode préféré de réalisation de l'invention, la cage comporte le trou tandis que la plaque comporte le téton.

Lorsque la cage comporte le trou tandis que la plaque comporte le téton, le trou sera avantageusement prévu sur la face latérale antérieure de la cage. Le téton sera pour sa part avantageusement prévu entre les trous présents vers chacune des extrémités de la plaque. Inversement, Lorsque la cage comporte le téton et que la plaque comporte le trou, le téton sera avantageusement prévu sur la face latérale antérieure de la cage tandis que le trou sera avantageusement prévu entre les trous présents vers chacune des extrémités de la plaque.

Le téton peut s'insérer et se bloquer de manière réversible dans ledit trou et permet la rotation de la plaque par rapport à la cage. Il pourra correspondre à une saillie de forme parallélépipédique, mais de préférence cylindrique ou inscrite sur un cylindre. Ces dernières permettent plus facilement la rotation de la plaque par rapport à la cage.

Les formes et dimensions relatives du téton et du trou seront telles que le téton peut être inséré dans le trou et bloqué en traction. Par exemple, un téton cylindrique pourra coopérer avec un trou de forme cylindrique mais aussi cubique ou de section hexagonale par exemple.

Dans un mode préféré de réalisation de l'invention, le téton possède un système de blocage dudit téton dans le trou, notamment réalisé au moyen d'une collerette en relief présente à l'extrémité du téton de préférence de forme cylindrique ou au moyen d'ergots présents à l'extrémité du téton ou encore au moyen d'un collet de préférence biseauté pour faciliter l'introduction du téton dans le trou, situé de manière intermédiaire sur la longueur du téton. Dans ce dernier cas, une gorge pourra être prévue dans le trou pour l'insertion dudit collet. La gorge aura alors une de ses parois inclinée pour permettre le retrait de la plaque par traction.

Selon un mode de réalisation de l'invention, le trou est fraisé en son extrémité interne pour permettre le retrait de la plaque par traction lorsque la collerette est présente à l'extrémité du téton.

Dans des conditions préférentielles de mise en oeuvre de l'invention, le téton est creux, notamment un cylindre creux, et possède des fentes longitudinales, lui conférant des propriétés d'élasticité. Ainsi, lors de l'introduction du téton dans le trou, les éléments de paroi du téton peuvent se rapprocher pour permettre l'introduction puis reprendre leur configuration d'origine pour permettre le blocage en fin d'introduction.

Selon encore un mode de réalisation de l'invention, le téton est prévu sur la plaque et possède dans sa partie interne un système de fixation, permettant d'accrocher un dispositif de démontage comme on le verra ci-après. Le système de fixation peut consister par exemple en une gorge et notamment en un filetage.

On peut aussi prévoir une cage et une plaque munis de trous, et une pièce de jonction ayant par exemple la forme d'une cheville de préférence cylindrique, agencée par exemple comme le téton.

La cage comporte avantageusement, sur au moins une des faces latérales, au moins une lumière lui permettant de se déformer lors des mouvements de la vie courante d'un individu de façon à transmettre à une greffe, située à l'intérieur de ladite cage, des contraintes mécaniques stimulant la croissance des ostéoblastes.

Les lumières situées dans les faces latérales gauche et droite sont avantageusement sensiblement symétriques et peuvent avoir une forme de fentes horizontales, alternées ou empilées ou obliques dans le cas de pluralité de fentes, une forme de trous jointifs ou espacés, de forme parallélépipédique ou de préférence circulaire, alignés ou non, ou une forme de trous reliés par une fente longitudinale. Ces lumières présentent la double fonction de permettre la vascularisation du greffon et de conférer à la cage les propriétés de déformabilité souhaitées. Dans un tel cas, la cage est de préférence réalisée en PEEK.

La présente demande a aussi pour objet un dispositif de démontage d'un système ci-dessus comportant un axe muni d'un manche, ledit axe possédant à une extrémité un dispositif d'accrochage de la plaque ou du téton, installé dans un tube comportant à une extrémité un pont muni de deux jambes espacées pour se placer de part et d'autre de la plaque et prendre appui sur la cage, ledit axe pouvant être déplacé longitudinalement dans le tube.

Selon un mode de réalisation, l'axe est coulissant et possède un levier à son extrémité opposée à celle munie d'un dispositif d'accrochage, levier qui, lorsqu'il est actionné, permet le déplacement longitudinal de l'axe et ainsi le déblocage du téton et le retrait de la plaque par simple traction.

Selon un autre mode de réalisation de l'invention, l'axe est fileté à une extrémité et coopère avec une butée ménagée sur le tube. En se vissant dans le filetage interne du téton, et bloqué en translation par le tube, l'axe permet d'opérer une traction pour débloquer le téton et retirer la plaque. Le pont peut être intérieurement fileté selon l'axe pour mieux guider ce dernier.

Selon encore un autre mode de réalisation de l'invention, l'accrochage de la plaque est obtenu en prévoyant des crochets à l'extrémité de l'axe pour crocheter la plaque.

Dans des conditions préférentielles de mise en oeuvre de l'invention, l'extrémité de l'axe opposée au manche est munie de filets, tandis que le téton possède un filetage interne permettant la coopération avec l'extrémité de l'axe. En coopération avec les « jambes » s'appuyant sur la cage, ce système permet par simple traction de débloquer le téton et retirer la plaque.

La présente demande a aussi pour objet un kit renfermant un système et un dispositif de démontage ci-dessus

La présente demande a encore pour objet un kit renfermant un système et un dispositif de démontage ci-dessus ainsi que les vis utiles au vissage de la plaque sur les vertèbres.

Le système de contention et de fusion intersomatique des vertèbres comprenant une cage et une plaque tel que décrit ci-dessus ainsi que son dispositif de démontage, objets de la présente invention, possèdent de très intéressantes propriétés et qualités.

Ce système permet notamment le retrait facilité de la plaque en laissant la cage en place, sans avoir à procéder préalablement par exemple à sa translation. De plus, rotatif, il permet au chirurgien d'installer librement la plaque sur les corps vertébraux.

Ces qualités sont illustrées ci-après dans la partie expérimentale. Elles justifient l'utilisation de ce système de contention et de fusion intersomatique des vertèbres et de son dispositif de démontage ci-dessus décrits, dans la chirurgie du rachis cervical.

Le système de contention de l'invention peut également comprendre deux ou plusieurs systèmes comprenant une cage et une plaque ci-dessus décrits, reliés entre eux par lesdites plaques se chevauchant ou non, deux par deux.

Les conditions préférentielles de mise en oeuvre des systèmes de contention et de fusion intersomatique des vertèbres et dispositifs de démontage ci-dessus décrits s'appliquent également aux autres objets de l'invention visés ci-dessus, notamment aux kits les renfermant.

L'invention sera mieux comprise si l'on se réfère aux dessins annexés sur lesquels
- la figure 1 représente une vue en perspective et coupe partielle du téton dans la loupe d'une cage et d'une plaque démontées.
- la figure 2 représente une vue en perspective de la cage et de la plaque montées.
- la figure 3 représente une vue en perspective d'un dispositif de démontage de la plaque.
- la figure 4 représente une vue en coupe du dispositif de démontage de la plaque.
- la figure 5 représente en perspective l'extrémité du dispositif de démontage de la plaque avant traction sur la plaque.
- la figure 6 représente en perspective l'extrémité du dispositif de démontage de la plaque après traction sur la plaque.

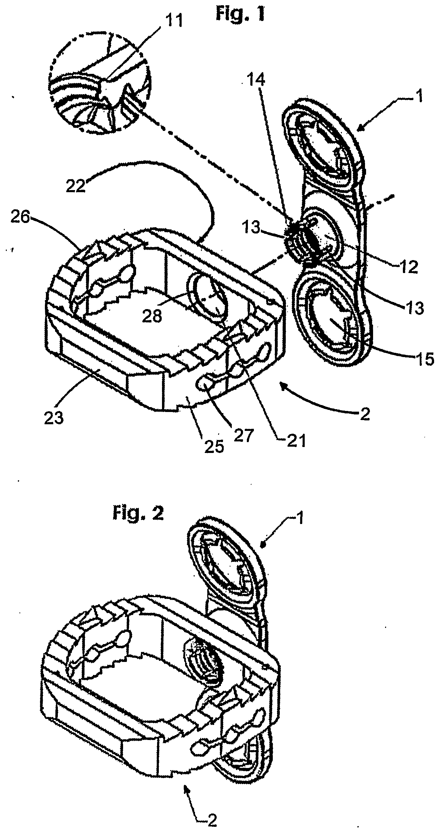

Les termes à signification directionnelles tels que « antérieur » et « postérieur », « haut » et « bas » ou « supérieur » et « inférieur » se réfèrent à l'orientation de la cage lorsqu'elle est implantée dans la colonne vertébrale à traiter. Ainsi, sur la figure 1, la paroi latérale antérieure est disposée en haut à droite et la paroi latérale postérieure est disposée en bas à gauche.

Comme on peut l'observer sur la figure 1, selon l'invention le système comprend une plaque 1 et une cage 2.

La cage 2 représentée sur la figure 1 est de forme générale parallélépipédique et symétrique par rapport à un plan longitudinal antéro-postérieur passant par l'axe général de la colonne vertébrale. La cage 2 comporte une paroi latérale antérieure 22 et une paroi latérale postérieure 23.

Les parois latérales antérieure 23 et postérieure 22 sont reliées entre elles par des parois latérales droite 25 et gauche 26.

La cage 2 est complètement ouverte vers le haut et vers le bas et ne comporte donc pas de paroi supérieure ou inférieure.

Dans la cage intervertébrale illustrée, les quatre parois latérales s'élèvent perpendiculairement à un plan transversal à l'axe de la colonne.

Les parois latérales droite 25 et gauche 26 sont munies de lumières allongées 27, d'axe parallèle à un plan transversal à l'axe de la colonne. Sur la figure 1, la forme de réalisation de la lumière comprend trois évidements cylindriques reliés par une fente diamétrale.

La cage 2 présente intérieurement un volume libre délimité par les parois latérales, destiné à être rempli par un greffon osseux.

La paroi latérale antérieure 22 de la cage 2 possède un trou cylindrique 21. Ce dernier comporte intérieurement un fraisage 28.

La plaque 1, installée perpendiculairement au plan général de la cage 2, possède une forme parallélépipédique aplatie généralement plane, arrondie à ses extrémités.

Elle est solidaire d'un téton cylindrique creux 12 muni d'entailles allongées 13 et d'un bourrelet 11 de diamètre légèrement supérieur à celui du trou 21 de la cage 2, mais inférieur à celui du fraisage 28. Les quatre entailles allongées 13 donnent aux quatre portions de cylindre ainsi formées une certaine élasticité permettant au téton creux 12 d'être introduit dans le trou 21 puis de s'y bloquer par coopération entre le bourrelet 11 et le fraisage 28.

Le téton creux 12 possède dans sa partie interne un filetage 14 destiné à coopérer avec le dispositif de démontage de la plaque décrit ci-après.

L'ancrage de la plaque 1 sur des vertèbres adjacentes, s'effectue par l'intermédiaire de vis (non représentées) traversant des trous 14 pratiqués à chacune des extrémités de ladite plaque 1 comme cela est visible sur la figure 1. Ces trous sont disposés sur la plaque 1 de part et d'autre du cylindre 12.

La figure 2 permet d'observer la plaque 1 et la cage 2 montées ensemble. Il est possible de faire pivoter relativement la plaque 1 et la cage 2, ce qui procure différents emplacements d'installation des vis de fixation.

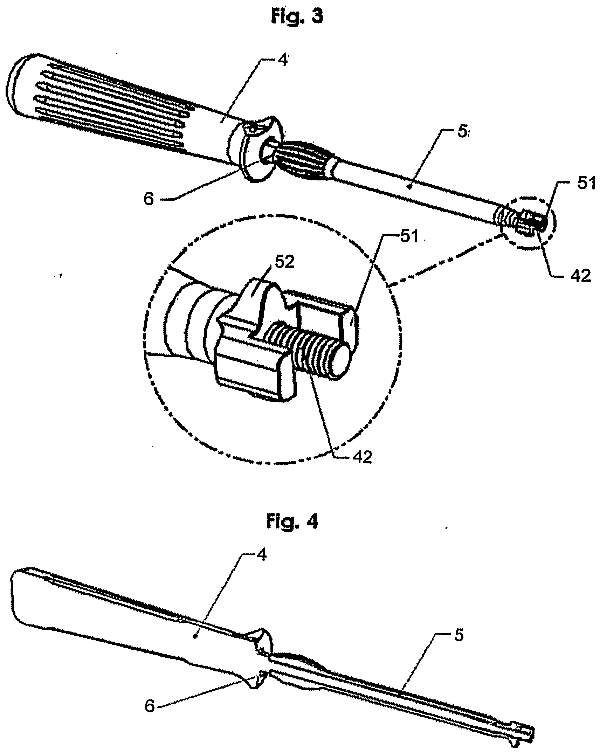

Sur la figure 3, on peut observer un dispositif de démontage selon l'invention. L'outil est composé d'un axe 4 et d'un tube 5.

Le tube 5 possède en une extrémité un pont 52 muni de deux jambes 51 et à l'autre un élargissement nervuré permettant de le maintenir par pincement entre des doigts par exemple. Les jambes 51 sont écartées d'au moins la largeur de la partie médiane de la plaque 1.

L'axe 4 est fileté 42 à une extrémité et comporte un manche à l'autre extrémité, constitué par un élargissement de l'axe 4.

Il traverse le tube 5 et vient en butée à l'extrémité 6 du tube 5 opposée au pont fileté 52.

Ainsi les jambes peuvent être mises en appui contre la cage de part et d'autre de la plaque, on peut engager l'extrémité filetée 42 de l'axe 4 dans le filetage interne du téton creux 12, et par vissage en tournant le manche, on obtient l'extraction de la plaque 1, sans avoir à la déplacer autrement, notamment par translation. Le vissage produit ainsi un déplacement longitudinal de l'axe 4 dans le tube.

Sur la figure 4 on peut observer le dispositif de démontage ci-dessus en coupe présentant l'axe 4, le tube 5 et la butée 6 qui leur est commune.

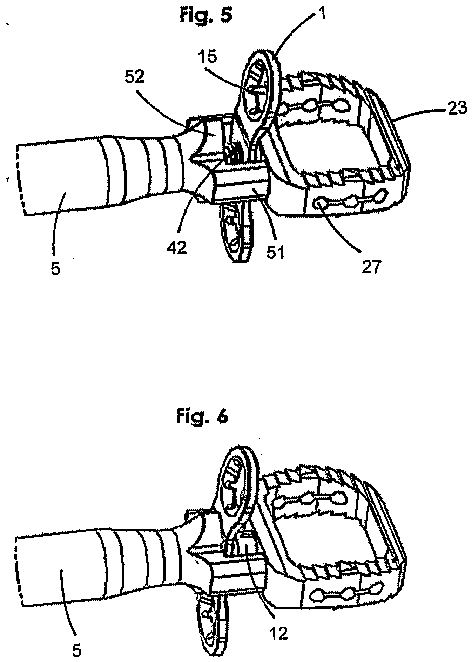

Sur la figure 5, on peut observer le dispositif de démontage de la plaque, dont les jambes 51 sont en appui sur la cage 2 de part et d'autre de la plaque 1. L'extrémité filetée 42 de l'axe 4 se visse dans la partie interne filetée du téton creux 12 installé sur la plaque 1.

Sur la figure 6, on peut observer le dispositif de démontage vissé sur la plaque. Par simple traction, le téton (12) a été débloqué et le retrait de la plaque 1 réalisé, tandis que la cage2 est restée en place.

La cage 2 représentée a été réalisée en PEEK. La plaque 1 a été réalisée en titane.

## Revendications

1. Un système de contention et de fusion intersomatique des vertèbres comportant au moins une cage et une plaque agencée perpendiculairement au plan général de ladite cage, en ce que ladite plaque (1) et ladite cage (2) possèdent un trou (21) et un téton (12) pouvant s'insérer et se bloquer de manière réversible dans ledit trou (21), ladite plaque (1) pouvant être ôtée par simple traction, **caractérisé en ce que** ledit téton (12) permet la rotation de la plaque (1) par rapport à la cage (2).

2. Un système selon la revendication 1, **caractérisé en ce que** le trou (21) est ménagé dans la cage et le téton (12) est installé sur la plaque (1).

3. Un système selon l'une des revendications 1 et 2, caractérisé en ce le téton (12) est de forme cylindrique.

4. Un système selon l'une quelconque des revendications précédentes, **caractérisé en ce que** le téton (12) est un cylindre creux possédant des fentes longitudinales.

5. Un système selon l'une quelconque des revendications précédentes, **caractérisé en ce que** le téton (12) possède un système de blocage dudit téton (12) dans le trou (21) prévu dans la plaque (1).

6. Un système selon l'une quelconque des revendications 1 à 5, **caractérisé en ce que** le téton (12) possède à une extrémité une collerette (11) en relief.

7. Un système selon l'une quelconque des revendications précédentes, **caractérisé en ce que** le téton (12) possède dans sa partie interne un système de fixation d'un dispositif de démontage.

8. Un système selon l'une quelconque des revendications 3 à 7, **caractérisé en ce que** le téton (12) possède dans sa partie interne un filetage.

9. Un kit comportant un système de contention et de fusion intersomatique des vertèbres tel que défini à l'une des revendications 1 à 8 et un dispositif de démontage pour un tel système, ledit dispositif comportant un axe (4) muni d'un manche et possédant à son extrémité un dispositif d'accrochage (42) de la plaque (1) ou du téton (12), installé dans un tube (5) possédant à une extrémité deux jambes (51) espacées pour se placer de part et d'autre de la plaque (1) et prendre appui sur la cage (2), ledit axe (4) pouvant être déplacé longitudinalement dans le tube (5).

10. Un kit selon la revendication 9, **caractérisé en ce que** l'axe (4) du dispositif de démontage est fileté (42) en son extrémité opposée au manche.

## Patentansprüche

1. System zum Halten und intersomatischen Fusionieren von Wirbeln, das mindestens einen Käfig und eine senkrecht zu der Hauptebene des Käfigs angeordnete Platte, wobei die Platte (1) und der Käfig (2) ein Loch (21) und einen Bolzen (12), der auf reversible Weise in das Loch (21) einführbar und verriegelbar ist, aufweisen, und die Platte (1) durch einfaches Ziehen entfernt werden kann, **dadurch gekennzeichnet, dass** der Bolzen (12) eine Drehung der Platte (1) relativ zu dem Käfig (2) zulässt.

2. System nach Anspruch 1, **dadurch gekennzeichnet, dass** das Loch (21) in dem Käfig vorgesehen ist und der Bolzen auf der Platte (1) installiert ist.

3. System nach einem der Ansprüche 1 und 2, **dadurch gekennzeichnet, dass** der Bolzen (12) eine zylindrische Form aufweist.

4. System nach einem der vorstehenden Ansprüche, **dadurch gekennzeichnet, dass** der Bolzen (12) ein Hohlzylinder mit Längsschlitzen ist.

5. System nach einem der vorstehenden Ansprüche, **dadurch gekennzeichnet, dass** der Bolzen (12) ein Verriegelungssystem für den Bolzen (12) in dem Loch (21) aufweist, dass in der Platte vorgesehen ist.

6. System nach einem der Ansprüche 1 bis 5, **dadurch gekennzeichnet, dass** der Bolzen (12) an einem Ende einen erhabenen Kragen (11) aufweist.

7. System nach einem der vorstehenden Ansprüche, **dadurch gekennzeichnet, dass** der Bolzen (12) in seinem inneren Abschnitt ein Befestigungssystem für eine Demontagevorrichtung aufweist.

8. System nach einem der Ansprüche 3 bis 7, **dadurch gekennzeichnet, dass** der Bolzen (12) in seinem inneren Abschnitt ein Gewinde aufweist.

9. Satz mit einem System zum Halten und intersomatischen Fusionieren nach einem der Ansprüche 1 bis 8 und einer Demontagevorrichtung für so ein System, wobei die Vorrichtung eine mit einem Griff versehene Achse (4) aufweist und an seinem Ende der Platte (1) oder des Bolzens (12) eine Kupplungsvorrichtung (42) aufweist, und in einem Rohr (5) installiert ist, das an einem Ende zwei Schenkel (51) aufweist, die so beabstandet sind, dass sie auf beiden Seiten der Platte (1) angeordnet sind und den Käfig (2) tragen, wobei die Achse (4) in Längsrichtung in dem Rohr (5) verlagert werden kann.

10. Satz nach Anspruch 9, **dadurch gekennzeichnet, dass** die Achse (4) der Demontagevorrichtung an seinen zum Griff gegenüberliegenden Enden mit einem Gewinde (42) versehen ist.

## Claims

1. A system for setting and intersomatic fusion of vertebrae, comprising at least one cage and one plate arranged perpendicular to the main plane of said cage, where that said plate (1) and said cage (2) have a hole (21) and a stud (12) which can be inserted and reversibly blocked in said hole (21), said plate (1) being able to be removed by simple traction, **characterized in that** said stud (12) permits the rotation of the plate (1) relative to the cage (2).

2. A system as claimed in claim 1, **characterized in that** the hole (21) is formed in the cage, and the stud (12) is fitted on the plate (1).

3. A system as claimed in one of claims 1 and 2, **characterized in that** the stud (12) is of cylindrical shape.

4. A system as claimed in any one of the preceding claims, **characterized in that** the stud (12) is a hollow cylinder with longitudinal slits.

5. A system as claimed in any one of the preceding claims, **characterized in that** the stud (12) has a system for blocking said stud (12) in the hole (21) provided in the plate (1).

6. A system as claimed in any one of claims 1 through 5, **characterized in that** the stud (12) has a raised flange (11) at one end.

7. A system as claimed in any one of the preceding claims, **characterized in that** the stud (12) has, in its inner part, a system for fixing of a disassembly device.

8. A system as claimed in any one of claims 3 through 7, **characterized in that** the stud (12) has a thread in its inner part.

9. A kit comprising a system for setting and intersomatic fusion of vertebrae such as is defined in one of claims 1 through 8, and a disassembly device for such a system, said device comprising a shaft (4) equipped with a handle and having, at its end, a device (42) for fastening the plate (1) or the stud (12), installed in a tube (5) which at one end has two spaced legs (51) for placing on either side of the plate (1) and for bearing on the cage (2), said shaft (4) being able to be displaced longitudinally in the tube (5).

10. A kit as claimed in claim 9, **characterized in that** the shaft (4) of the disassembly device is threaded (42) at its end remote from the handle.
